# EUROPEAN PATENT APPLICATION

(11) **EP 2 103 252 A1**
(43) Date of publication of application: **23.09.2009**
(21) Application number: 08200010.0
(22) Date of filing: 20.03.2008
(51) Int. Cl.: A61B 5/00, G06F 19/00, A61B 5/11

(54) **Improvements relating to monitoring apparatus**

(71) Applicant: BAE Systems PLC, London, Greater London SW1Y 5AD (GB)
(72) Inventor: The designation of the inventor has not yet been filed
(74) Representative: BAE SYSTEMS plc Group IP Department

(57) **Abstract**

Monitoring apparatus comprises at least one experiment node at which, in use of the apparatus, a user participates in a study, and a control node. The experiment node comprises communication means operable to communicate with the user, user input means operable to record the response of a user to a prompt, user interaction means operable such that the user can interact with the experiment node, and means to record data relating to such user interactions. The control node is remote from the experiment node and in communication therewith to receive information comprising user responses and recorded user interaction data therefrom. The control node is also operable to configure the at least one experiment node to execute a plurality of human factors tests on the participant at predetermined times. The monitoring apparatus also comprises co-ordination means operable to co-ordinate the information with the predetermined times.

## Description

This invention relates to improvements to monitoring apparatus. More particularly, this invention relates to the real-time capture and analysis of objective and subjective measures which are used to assess human performance, either individually or as teams. Such measures are collectively referred to as human factors data in the present specification.

Human factors data may include results from a number of experiments, or tools; may include physiological measurements; and may include subjective data-gathering tools such as electronic questionnaires, mental workload tests, and situation awareness tests. Examples of such tools include heart rate and body temperature measurements; eye tracking; the NASA Task Load Index (hereinafter referred to as NASA TLX), a subjective mental workload assessment tool; and the Crew Awareness Rating Scale (hereinafter referred to as CARS), a subjective awareness rating tool.

Generally, human factors tools and experiments are designed to gather information, or improve understanding of, human performance, behaviour, and interactions. Such tools can be used to help improve design of, for example, aircraft cockpits and train cabs; or to study and assess the effects of training. Many such tools, including those examples given above, are known in the art. However, these tools are most normally used in an isolated manner, functioning independently of other systems and of each other. This leads to difficulties in analysing the results from a number of different tests performed during the course of a study.

It is an aim of the present invention to overcome or at least mitigate some of the above-mentioned problems.

In accordance with a first aspect of the present invention, there is provided monitoring apparatus comprising: at least one experiment node at which, in use of the apparatus, a user participates in a study; the at least one experiment node comprising communication means operable to communicate with the user, user input means operable to record the response of a user to a prompt, user interaction means operable such that the user can interact with the experiment node, and means to record data relating to such user interactions; a control node remote from the at least one experiment node and in communication with the at least one experiment node to receive information comprising user responses and recorded user interaction data from said at least one experiment node, and to configure the at least one experiment node to execute a plurality of human factors tests on the participant at predetermined times; and co-ordination means operable to co-ordinate the information with the predetermined times.

Such monitoring apparatus enables analysis of data, after a study, to be performed without the problems associated with prior-known systems. A number of human factors tests applied to a subject can be co-ordinated using such apparatus. Data from diverse tests and locations resulting from one study is integrated into a single, co-ordinated set of results using the present invention. This represents a considerable improvement over prior known monitoring systems for use in human factors tests. Co-ordination is particularly advantageous where there is a plurality of experiment nodes.

It is anticipated that the monitoring apparatus of the present invention will find application in human factors studies performed using simulators. In such cases, in use of the apparatus, the user participates in a study comprising a simulation. The at least one experiment node may then comprise simulation means configurable by the control node. Using monitoring apparatus in accordance with the present invention, it is possible to configure the simulation, such that the user's response to a variety of situations and events can be monitored. Moreover, the experiment controller is able to adapt the simulation in real time, in dependence on the simulation user's performance and response to events as they unfold in the simulation. However, the monitoring apparatus of the present invention is not limited to use in simulated environments, and may be used in real situations; for example, to perform a plurality of human factors tests on the driver of a tank during an exercise.

The means to record data relating to user interactions may comprise a video camera, and the user interaction data may comprise live video feed of the user participating in the study. The means to record data relating to user interactions may also comprise head tracking equipment or sound recording devices. A particular advantage of the use of video feed is that it can also be used to facilitate control of the experiment.

The experiment node may further comprise physiological monitoring means operable to record physiological data for the participant; and wherein the information comprises the physiological data. One of the plurality of human factors tests may be a physiological test. Human factors physiological data may include, for example, data on heart rate, or eye tracking.

There may be an observer node in communication with the at least one experiment node, at which node observations of the participant at the at least one experiment node can be made and recorded, and which observation node is in communication with the control node to transmit recorded observations to the control node. Expert observations can then be recorded, in real time, along with data from the various human factors tests applied at the experiment node. Conveniently, the co-ordination means is operable to co-ordinate the recorded observations with the predetermined times and the information. In one particular embodiment, the observer node receives live video feed from the at least one experiment node. Alternatively, the observer node may receive a screen feed from a simulation to indicate how the user is performing in a simulation.

Each of the experiment nodes, the control node and the observation node may be linked together via a network.

The plurality of human factors tests may include both an objective and a subjective test. One example of a subjective test is a subjective workload assessment, which assessment is performed on a prompt communicated to the user by the experiment node, the user providing a response indicative of the users' instantaneous workload. The experiment node may communicate the response indicative of the user's instantaneous workload in combination with video feed of the user recorded for a period of time prior to the prompt. The combination of video feed with the recorded response for a subjective workload assessment enhances the workload test since missing responses, where the user is prompted for an assessment but fails to provide the assessment, can be better accounted for. For example, the provision of video feed may indicate that a missed response was due to a particularly high workload, or simply due to the user not noticing the prompt. The period is preferably in the range between 1 second and 1 minute, or more preferably 4 seconds. Such time scales are selected to balance the need for a reasonable amount of video feed to enable an experiment controller to assess why a response was missed with the need to minimise the amount of information it is necessary to communicate between the experiment node and the control node.

The above and further features of the invention are set forth in the appended claims, and will be described in detail hereinafter with reference to various exemplary embodiments which are illustrated in the accompanying drawings, in which:
Figure 1 is a schematic illustration of a first embodiment of the invention; and
Figure 2 is a schematic illustration of a second embodiment of the invention.

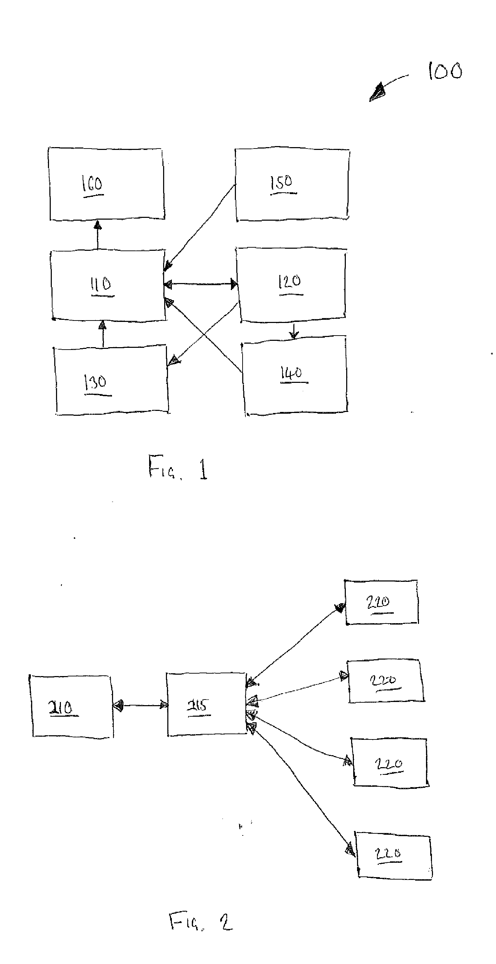

Monitoring apparatus 100 in accordance with a first embodiment of the invention is illustrated in Figure 1. Monitoring apparatus 100 is particularly suited to the monitoring of participants engaging in human factors experiments, and to the retrieval and collation of data resulting from such experiments. Such experiments can result in a large amount of diverse types of data being collected, and benefit from the involvement of experts in various fields. The monitoring apparatus 100 was developed with the aim of facilitating such experiments.

Monitoring apparatus 100 comprises a control node 110, an experiment node 120, an observation node 130, a logger node 140, a programming interface node 150, and an analysis node 160. Each node comprises a standard computer having a display device and user interaction means, such as a keyboard, mouse, or voice recognition means. These nodes are in mutual communication through a TCP/IP network as illustrated in Figure 1. Control of the nodes and their communication is achieved using software written in the JAVA programming language.

The subject of the experiment takes part in a simulation at the experiment node 120, at which node a number of human factors tests will be applied, including, for example, questionnaires to subjectively assess the participant's workload or physiological measurements to determine the participant's physiological reaction to the simulation. The control node 110 controls and configures the experiment node 120. The control node is operable to start and to stop the running of a simulation, to trigger the start of events within the simulation, and to determine when, or at what intervals, human factors tests are applied. Thus, an experiment controller at the control node may choose to prompt the participant at the experiment node to self assess his or her workload once every minute throughout the running of the simulation, and may prompt physiological measurement apparatus to record the participants heart rate once every minute throughout the simulation. The controller may also wish to alter the rate at which measurements are taken, for example if the participant is experiencing a particularly high workload due to a particular simulation event. The control node also comprises co-ordination means to co-ordinate the data received from the experiment, for example by the addition of a time stamp to the data.

The observation node 130 and the logger node 140 facilitate the monitoring and recording of the experiment in progress. The observation node 130 enables the performance of the participant to be monitored by an expert observer able to provide an objective assessment of the participant's performance. For example, where the experiment node 120 subjects the participant to a flight simulator, the expert observer may be a pilot with several years of flying experience. The observation node 130 receives, from the experiment node 120, data reflecting the participant's performance. Such data may include a video feed of the participant, or a screen feed replica of the participant's view of the simulation, enabling the expert observer to view the participant's actions in response to the simulation. Such data may also include data relating to the participant's use of the control systems - for example, where the simulation is a flight simulation, the observer may receive data relating to the participants use of the joystick, or other flight controls. The observation node 130 allows data input from the expert observer in the form of comments, which comments are recorded and communicated to the control node 110. The logger node 140 records the progress of the simulation in a standard language such that the sequence of events occurring in the simulation is recorded in a format that can be compared to other simulations. One particular such format is the Distributed Interactive Simulation.

The programming node 150 and the analysis node 160 further enhance the operability of the monitoring system, although, in contrast to the control node 110, experiment node 120, observation node 130 and logger node 140, these nodes are not operable in real time to modify the progress of a particular experiment. The programming node 150 provides an interface with the system such that non-standard human factors tools, in addition to those stored at the control node, can be developed in order to enhance the available toolset. Furthermore, the programming node enables interaction with the hardware of the experiment node, such that, for example the participant's use of the hardware can be monitored. The analysis node receives experimental data from the control node and enables detailed analysis of the experimental data at a convenient time after the experiment has been completed.

By separating the functional components of the monitoring apparatus in this manner, it is possible for the various nodes to be located remotely from one another, increasing the flexibility with which the experiment can be carried out. An expert observer and subject can be located separately, and an experimenter controlling the experiment can be again located remotely.

By way of example, the monitoring apparatus 100 has been used for the monitoring of a trainee pilot participating in a landing exercise on a flight simulator. The trainee pilot sits at the experiment node 120. The experiment controller is located at the control node 110. The experiment 120 and control 110 nodes are remote from one another: they may, for example, be located in separate countries. The controller selects which tests should be used to assess the performance of the trainee pilot as the landing exercise is carried out. In the present example, an instantaneous self assessment (ISA) test for workload is presented to the trainee pilot. This test asks the pilot to respond to subjectively assess his workload during the exercise, using a numeric rating of between 1 and 5 representing different levels of workload (1 representing a low workload, and 5 representing a high workload). The experiment node prompts the user to provide an assessment is requested at one minute intervals during the exercise. The results of the assessment are combined with video-feed of the trainee pilot in the four seconds around the prompt, recorded using a video camera at the experiment node. Thus, the three seconds before the prompt, and the one second immediately following the prompt, may be recorded. Recording the trainee pilot's actions around the time at which the prompt is given, on video, enables missed responses to be interpreted. For example, if the trainee pilot is about to land, his workload may be very high, and he may therefore not have the time to provide a workload assessment. On the other hand, it may be that the pilot is distracted by a concurrent test, or that the pilot simply fails to notice the prompt. Such information enables an experiment controller to better interpret the results of the workload assessment. Thus, the data recorded from the subjective workload assessment comprises a database of the responses, the times at which the responses and prompts were given, and a video recording of the period of time around each prompt. The trainee pilot's heart rate is also measured during the exercise.

The controller also selects events that may occur during the landing exercise, and can direct the participant to answer specific electronic questionnaires concerning that event immediately subsequent to that event. The management of these various tools is undertaken by the controller at the control node and can be achieved through use of a scripting language. Thus, to instigate the simulation of a fire in the cabin during the trainee pilot's landing exercise and subjectively assess the performance or workload of the trainee pilot during that event, the script would be:
After 5 minutes participant A will receive a "Fire Detected" message.
After 6 minutes participant A will receive an electronic questionnaire relating to the fire.

The use of such scripts enables the experiment to be managed more effectively, and enables the experiments to be repeated with additional participants. The addition of such an event to the simulation exercise allows the performance of the pilot under duress to be assessed. The logger node 140 records such events in a standard format for future reference, such that the experiment can more easily be compared to simulation exercises performed using different monitoring apparatus.

An expert observer, in the present example an experienced pilot, is located at the observation node 130. The observer sees a video feed of the trainee pilot landing the simulated aircraft and is able to comment on the trainee pilot's performance. Such comments are either recorded in text input directly by the expert, or by direct speech input using voice-over-internet-protocol, although it is anticipated that direct speech input could also be used. The expert's comments are logged and recorded at the controller 110, along with a time stamp to indicate at what time the comment was made. The time stamp enables the expert's comments to be better linked to events occurring in the simulation. It is to be noted again that the expert can be remotely located from both the participating trainee pilot, and the experiment controller, further enhancing the flexibility of the apparatus. Such remoteness is particularly useful when it is desired to monitor a number of simulation participants at the same time: one expert can comment on the performance of a number of participants from one location.

During the simulation, the experiment controller is able to monitor the results of the ISA workload tests and heart rate measurements, and comments from the expert observer from the control node 110. Once the experiment is complete, the results are uploaded to separate analysis node 160, where more detailed analysis of the results can be performed, freeing the control node to perform further experiments. It is to be noted that, on upload to the analysis node 160, the results are already synchronised such that comparisons of, for example, heart rate and workload, can be made without recourse to further data manipulation.

Monitoring apparatus 200 in accordance with a second embodiment of the invention is illustrated in Figure 2. The monitoring apparatus 200 comprises a control terminal 210, central filestore and database 215, and a plurality of experiment nodes 220. The control terminal 210 and central filestore 215 in combination perform those function performed by the control node of the first embodiment 100. In the second embodiment 200, there is a plurality of experiment nodes 220, allowing a number of participants to take part in experiments simultaneously. The participants may, for example, be taking part in a multi-role simulation. The co-ordination of the data performed by the control terminal 210 is particularly advantageous in such cases, where a plurality of experiment nodes generate data. It is also particularly advantageous for the control terminal to be operable to start and to stop the running of a simulation at each of the plurality of experiment nodes, since the study controller, from the control terminal, can ensure that each participant, at each experiment node 210, is ready to start the simulation. Such a check can be readily performed where live video feed is transmitted from the experiment nodes to the control terminal. Monitoring apparatus 200 may be used in a similar manner to the monitoring apparatus 100 of the first embodiment. The separation of control terminal 210 and central filestore 215 reduces the data storage requirements for the control terminal, such that it can be more mobile.

The second embodiment of the invention may therefore be useful, for example, in actual tests of, for example, a plurality of armoured vehicles: an experiment controller may be able to observe the motion of the armoured vehicles in situ via wireless links to experiment nodes on each armoured vehicle, and control a developing experiment, for example by issuing instructions as to the goals of each armoured vehicle in the scenario, and by determining which measurements and human factors toolsets should be applied. The drivers of the vehicles can be monitored physiologically by head-tracking measurement, or heart rate measurement, by video monitoring, and by the provision of electronic questionnaires to assess workload, as in the above example.

It is to be noted that the embodiments described herein are in all respects exemplary. Further embodiments are envisaged. Moreover, variations and modifications to the described embodiments are possible without departing from the scope of the invention, which is defined in the accompanying claims. For example, it will be clearly understood that, whilst it has been described that only one expert observer be present at one observer node, it is possible to provide several observer nodes, with an expert observer at each node. The adaptability of the monitoring apparatus to such alterations, whilst maintaining a co-ordinated set of data, is a key advantage of the monitoring apparatus over prior known systems. Moreover, it will be noted that, whilst in the above it has been described that the control node and observation node (for example) are separate, it will be possible to integrate these functions at one location, or at one node. In order for the benefits of the present invention to be realised, it is only necessary to have two terminals to perform the control and experiment node functions. Functions such as are, in the above, described to be separate nodes can be integrated with other nodes. For example, the observer node can be integrated with the control node. In a similar manner, the programming node can be implemented at the experiment node, such that equipment used to record physiological measurements can be integrated with the monitoring apparatus. Furthermore, whilst, in the above, it has been described to record four seconds of video footage in association with each prompt in a subjective workload assessment, it will be appreciated that, in some embodiments of the invention, it may be preferable to record a longer or shorter length of video footage. Where there are limits to the amount of data that can be stored of transmitted over a network link, it may be preferable to record a shorter period of time, for example 1 second. Such shorter periods may also be appropriate where the prompt is given via a head-up display. However, where there are not such stringent limits to data storage or transmission capabilities, it may be preferable to record a longer period of video footage. Such longer periods may be particularly appropriate where the prompt is given via a screen to the side of the participant, such as on a tablet PC. Those skilled in the art will appreciate that the length of time recorded should be selected in dependence on the manner in which the prompt is given. It may also be preferred to record a period of time leading up to the prompt, rather than a period of time around the prompt.

It is also to be clearly understood that any feature described above in relation to any one embodiment may be used alone, or in combination with other features described, and may also be used in combination with one or more features of any other of the embodiments, or any combination of any other of the embodiments.

## Claims

1. Monitoring apparatus for a human factors study, the apparatus comprising:
at least one experiment node at which, in use of the apparatus, a user participates in a study; the at least one experiment node comprising communication means operable to communicate with the user, user input means operable to record the response of a user to a prompt, user interaction means operable such that the user can interact with the experiment node, and means to record data relating to such user interactions;
a control node remote from the at least one experiment node and in communication with the at least one experiment node to receive information comprising user responses and recorded user interaction data from said at least one experiment node, and to configure the at least one experiment node to execute a plurality of human factors tests on the participant at predetermined times; and
co-ordination means operable to co-ordinate the information with the predetermined times.

2. Monitoring apparatus as claimed in claim 1, wherein, in use of the apparatus, the user participates in a study comprising a simulation, and wherein the at least one experiment node comprises simulation means configurable by the control node.

3. Monitoring apparatus as claimed in claim 1 or claim 2, wherein the means to record data relating to user interactions comprises a video camera, and wherein the user interaction data comprises live video feed of the user participating in the study.

4. Monitoring apparatus as claimed in any one of claims 1 to 3, wherein the experiment node further comprises physiological monitoring means operable to record physiological data for the participant; and wherein the information comprises the physiological data, and wherein one of the plurality of human factors tests is a physiological test.

5. Monitoring apparatus as claimed in any preceding claim comprising a plurality of experiment nodes.

6. Monitoring apparatus as claimed in any preceding claim, further comprising an observer node in communication with the at least one experiment node, at which node observations of the participant at the at least one experiment node can be made and recorded, and which observation node is in communication with the control node to transmit recorded observations to the control node.

7. Monitoring apparatus as claimed in claim 6, wherein the co-ordination means is operable to synchronise the recorded observations with the predetermined times and the information.

8. Monitoring apparatus as claimed in claim 6 or claim 7 when dependent on claim 6, wherein the observer node receives live video feed from the at least one experiment node.

9. Monitoring apparatus as claimed in claim 3 wherein plurality of human factors tests includes a subjective workload assessment, which assessment is performed at a prompt communicated to the user by the experiment node, the user providing a response indicative of the users' instantaneous workload, and wherein the experiment node communicates the response indicative of the user's instantaneous workload in combination with video feed of the user recorded for a period of time prior to the prompt.

10. Monitoring apparatus as claimed in claim 19, wherein the period is in the range between 1 second and 1 minute.
